(19) Europäisches Patentamt / European Patent Office / Office européen des brevets

(11) **EP 2 334 186 B1**

(12) **EUROPEAN PATENT SPECIFICATION**

(45) Date of publication and mention
of the grant of the patent:
**02.01.2013 Bulletin 2013/01**

(51) Int Cl.:
*A01N 49/00* (2006.01)   *A01N 55/10* (2006.01)
*A01P 7/00* (2006.01)

(21) Application number: **09741212.6**

(22) Date of filing: **21.09.2009**

(86) International application number:
**PCT/EP2009/006800**

(87) International publication number:
**WO 2010/031584 (25.03.2010 Gazette 2010/12)**

(54) **USE OF VITAMIN E OR ITS ESTERS FOR THE CONTROL OF ECTOPARASITES**

VERWENDUNG VON VITAMIN E ODER SEINEN ESTERN FÜR DIE BEKÄMPFUNG VON EKTOPARASITEN

UTILISATION DE LA VITAMINE E OU DES ESTERS DE CELLE-CI POUR LA LUTTE CONTRE LES ECTOPARASITES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **22.09.2008 IT MI20081675**

(43) Date of publication of application:
**22.06.2011 Bulletin 2011/25**

(73) Proprietor: **BIO.LO.GA. S.r.l.**
**31015 Conegliano (TV) (IT)**

(72) Inventor: **PANIN, Giorgio**
**I-45100 Rovigo (IT)**

(74) Representative: **Ferreccio, Rinaldo**
**Botti & Ferrari S.r.l.**
**Via Cappellini, 11**
**20124 Milano (IT)**

(56) References cited:
**EP-A1- 0 881 882     EP-A1- 0 998 943**
**WO-A1-01/09000     WO-A1-98/10793**
**US-A- 4 654 328     US-A1- 2003 202 997**
**US-A1- 2008 193 387**

- **"Dedraflow 5.1 - Technical Data Sheet (Experimental); Ref: DF017" INTERNET CITATION 1 June 2008 (2008-06-01), XP007910430 Retrieved from the Internet: URL: http://www.creationscouleurs.com/tds/e _tds_ df51.pdf> [retrieved on 2009-11-04]**
- **BURGESS IAN F ET AL: "Treatment of head louse infestation with 4% dimeticone lotion: randomized controlled equivalence trial" BMJ. BRITISH MEDICAL JOURNAL, LONDON, GB LNKD- DOI:10.1136/BMJ.38497.506481.8F, vol. 330, no. 7505, 1 January 2005 (2005-01-01), pages 1423-1425, XP009127674 ISSN: 0959-8146**

Remarks:
The file contains technical information submitted after the application was filed and not included in this specification

**Description**

Field of the invention

The present invention relates to the non-therapeutic use of a composition containing vitamin E or an ester thereof for the control of ectoparasites, in particular lice and nits.

Background of the invention

Pediculosis is the technical term for infestation by lice, small white-greyish coloured parasites which live on humans and suck their blood. They are small (from one to three millimetres) in size and lay their eggs by attaching them to the shaft of hairs, on which they can easily move thanks to their hooked feet.

Louse infestations are frequent in both rich and developing countries and there is no direct correlation between personal hygiene, the state of household cleanliness and the distribution of these parasites. This is because transmission occurs by direct contact with people who are already infested or by exchanging clothing or personal effects, such as pillows, hats, scarves or combs.

There are three different species of lice: the head louse (*Pediculus capitis*), the body louse (*Pediculus humanus*) and the pubic louse (*Phthirus pubis*). The first is the most widespread and is virtually indistinguishable from the body louse, which is now somewhat rare.

The infestation is manifested by irritation and intense itching in the affected area, which in turn causes dermatitis, impetigo and other similar conditions due to staphylococci.

The eggs, known as nits, are light coloured and are approx. 1 mm long. The female head louse deposits nits at the hair roots using a kind of very strong glue. The eggs mature and hatch in 7 days. Over the course of one month, the various species may lay from 80 to 300 eggs on the host. When an infestation is found, use is generally made of insecticidal products. There are many commercially available products formulated as sprinklable powders, shampoos, lotions or sprays. The most widely used active ingredients are pyrethrum extract or synthetic pyrethroids such as tetramethrin or an organophosphorus insecticide such as malathion. A fine-toothed comb is another essential means for removing the eggs and lice killed by the insecticide. A second application of insecticide is generally required 7-10 days after the first in order to kill any lice which have hatched out of their eggs since the first treatment.

The above-mentioned insecticides may, however, have toxic effects which are sometimes more hazardous than the pediculosis itself. Furthermore, lice and nits may develop resistance to such insecticides. Patent application GB 1 604 853 suggested using linear siloxane polymers, such as for example dimethicone, with a viscosity of less than 20,000 centistokes for the control of ectoparasites, such as lice and their eggs.

Patent application EP 0 191 543 describes a method for the control of insects and spiders by using compounds containing silicon, such as for example cyclic siloxanes, silanes, linear siloxanes etc..

Patent application EP 1 215 965 describes the use of a composition comprising, as active ingredient, a volatile siloxane and a non-volatile siloxane at least one of which is different from a linear alkyl or aryl siloxane with a viscosity of less than 20,000 centistokes, for the control of arthropods.

Patent application DE 196 22 190 discloses arthropodicidal compositions based on cyclic polysiloxanes in admixture with active compound with arthropodicidal activity. It is stated in this application that tetrameric or pentameric cyclic polysiloxanes are known to be effective as insecticides, but they have an insufficient duration of activity. In this application it is thus proposed to add to such cyclic polysiloxanes known arthropodicides, such as organophosphus compounds, pyrethroids, carbamates etc., in order to increase the activity duration.

However, if on the one hand the duration of activity is increased, on the other hand, the toxicity and resistance problems mentioned before in connection with the use of insecticides are still displayed by the composition according to DE 196 22 190.

The composition according to DE 196 22 190 may contain several additives, among which antioxidant agents; tocopherol is mentioned among the latter agents, but nothing is said about any activities of tocopherol other than an antioxidative activity.

A treatment for head louse infestations using a 4% lotion of dimethicone has recently been described (British Medical Journal 2005; 330; 1423-) and its efficacy relative to phenothrin 0.5% liquid was evaluated. It has been shown that a louse infestation may be treated with dimethicone, which would seem to be less irritant that pre-existing treatments and has a physical action on the louse which ought not to be affected by resistance to neurotoxic insecticides. The dimethicone lotion used in the clinical testing contained 4% of dimethicone in a volatile cyclomethicone base.

As explained in the above-mentioned article, the lotion is applied uniformly onto the hair and scalp, after which, once the cyclomethicone has evaporated, the product dries and only the active ingredient, composed of the dimethicone, remains.

Brief description of the invention

The problem underlying the present invention was that of providing a composition capable of controlling ectoparasites such as for example lice, which exhibits a level of efficacy comparable to or better than that exhibited by conventional insecticide-based compositions, accompanied by excellent tolerability characteristics and negligible if not zero toxicity. Such a problem has been solved by the non-therapeutic use of a a composition consisting of vitamin E or an ester thereof selected among the group consisting of esters of tocopherol with acetic, linoleic, oleic, palmitic or succinic acid, and a solvent selected from the group consisting of volatile siloxanes, hydrogenated polyisobutene, hydrogenated polydecene and mixtures of hydrogenated polyisobutene and/or hydrogenated polydecene with hydrogenated polyolefines, in the control of ectoparasites.

A "volatile siloxane" is taken to mean a siloxane which has a measurable vapour pressure at 25°C and generally has a viscosity of less than 50 centistokes, together with a boiling point of less than 300°C.

The above-mentioned mixtures of hydrogenated polyisobutene and/or hydrogenated polydecene with hydrogenated polyolefines are preferably mixtures of hydrogenated polyisobutene and hydrogenated polydecene with hydrogenated C6-C14 polyolefines, such as the product Dedraflow® 5.1 marketed by the company Cosmetic Innovations & Technologies (France).

The volatile siloxane is preferably selected among cyclomethicone pentamer, cyclomethicone tetramer, cyclomethicone hexamer, hexamethyldisiloxane and mixtures thereof. It is particularly preferred to use cyclomethicone pentamer.

The vitamin E may be used as d-$\alpha$-tocopherol or as a mixture of the two d and 1 enantiomers of $\alpha$-tocopherol or as a mixture of other tocopherols ($\beta$, $\gamma$, $\epsilon$, $\xi$, $\eta$) or as tocotrienols. It is preferred to use $\alpha$-tocopherol acetate.

In one embodiment of the non-therapeutic use according to the invention, the composition comprises from 1% to 90% of vitamin E or a derivative thereof. Suitable compositions for the present invention comprise from 1 to 30% of vitamin E or a derivative thereof.

Other suitable ranges for the content of vitamin E or of a derivative thereof are: from 1 to 20%, from 1 to 10%, from 1 to 5%, from 5 to 70%, from 5 to 30%, from 5 to 20%, from 5 to 10%, from 10 to 70%, from 10 to 30% and from 10 to 20%.

According to one aspect of the non-therapetic use of the present invention, the composition essentially consists of vitamin E or an ester thereof and cyclomethicone, in particular cyclomethicone pentamer. Said composition is preferably delivered by means of a spray delivery device.

Said spray delivery device may be composed of a "no-gas" atomizer or of a spray can containing the composition according to the invention and a propellant such as a saturated hydrocarbon, for example propanol or butanol, or a hydrofluorocarbon (HFC).

According to one aspect of the present invention, the spray delivery device for the composition comprises a collapsible container which is in turn enclosed in a spray can filled with a pressurized fluid which causes said composition to be delivered in finely atomized form.

Saturated hydrocarbons, such as propanol or butanol, or fluorinated hydrocarbons (HFC) may be used as the pressurized fluid, but it is preferred to use nitrogen, carbon dioxide and conveniently air. Spray delivery devices composed of a can containing inside it a collapsible container are described for example in patent application FR 2 436 085. It has proved particularly convenient for the purposes of the present invention to use a spray device offered for sale by the company COSTER TECNOLOGIE SPECIALI SPA, ITALY.

Using the device in question, it is possible to apply the composition in finely atomized form resulting in the creation of a film having appreciable bioadhesive properties towards the hair and scalp, so promoting the action of the active ingredient.

According to another aspect of the present invention, a composition for the non-therapeutic use according to the invention is in the form of a hydrophobic gel and comprises vitamin E or an ester thereof, selected among the group consisting of esters of tocopherol with acetic, linoleic, oleic, palmitic or succinic acid, a volatile silicone, selected from the group comprising cyclomethicone pentamer, cyclomethicone tetramer, cyclomethicone hexamer, hexamethyldisiloxane and mixtures thereof, and hydrogenated castor oil.

According to an embodiment of the present invention, the above-stated composition contains from 1 to 70% of vitamin E acetate, from 20 to 90% of the above-stated volatile silicone and from 7 to 13% of hydrogenated castor oil.

In an other aspect, the above composition comprises from 1 to 50% of vitamin E acetate, from 40 to 70% of the above-stated volatile silicone and from 7 to 13% of hydrogenated castor oil.

Other suitable ranges for the content of vitamin E or of a derivative thereof are: from 5 to 70%, from 10 to 70%, from 20 to 70%, from 5 to 50%, from 10 to 50%, from 20 to 50%.

Cyclomethicone pentamer is preferred as the volatile silicone.

The composition may conveniently also contain from 7 to 15% of an oily component selected from the group comprising vegetable oils and esters of fatty acids such as octyl palmitate, isopropyl myristate, ethyl oleate and mixtures thereof, together with from 2 to 3% of dimethiconol.

By the non-therapeutic use according to claim 1, it has proved possible to achieve complete control of human ectoparasite infestations, and in particular of lice, without toxic or irritant effects.

In one aspect of the non-therapeutic use of the present invention, the above composition is delivered by means of a spray delivery device, which, in one embodiment, comprises a collapsible container, which is in turn enclosed in a spray can filled with a pressurized fluid that causes the composition to be delivered in finely atomized form.

Efficacy has proved to be superior not only to compositions based on conventionally used insecticides but also to lotions based on dimethicone.

In the case of louse infestations, use of the composition with the assistance of a spray delivery device has proved particularly advantageous thanks to the great uniformity of distribution achieved on the hair and scalp.

Detailed description

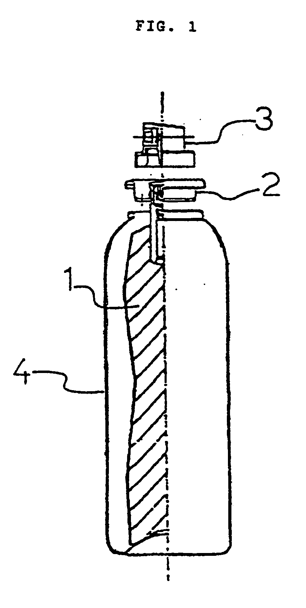

The present invention will be described in greater detail with reference to some examples which are provided by way of non-limiting illustration and also with reference to the single appended drawing (Fig. 1).

Said drawing is a schematic diagram of a spray device which comprises a collapsible container 1 containing the above-described composition connected to a valve 2 on which a delivery device 3 acts; the collapsible container 1 is in turn enclosed within a can 4, with the valve 2 crimped onto the edge of the can 4, and the space between the internal wall of the can 4 and the container 1 is filled with a pressurized gas.

EXAMPLE 1

| d,l-alpha-Tocopherol acetate | 20% |
|---|---|
| Cyclomethicone pentamer | 80% |

The above-stated percentages, and those which will be shown below, are percentages by weight relative to the total weight of the composition.

Once the collapsible container 1 has been inserted in the can 2 and the valve 2 has been crimped onto the edge of the can 4, the space between the container 1 and the internal wall of the can 4 was filled with air at 10 atmospheres. At this point, the solution obtained by dispersing the alpha-tocopherol acetate in the cyclomethicone was introduced into the collapsible container 1 by methods known in the art. When a non-esterified tocopherol or a tocotrienol is used as the vitamin E component, it is advisable introducing the relevant cyclomethicone solution into the collapsible container under a stream of an inert gas, such as nitrogen.

EXAMPLE 2

| Cyclomethicone pentamer 245 | 39.5% |
|---|---|
| Vitamin E acetate | 30.0% |
| Hydrogenated castor oil | 10.5% |
| Octyl palmitate | 10.0% |
| Cyclomethicone/dimethiconol 8:2 | 10.0% |

525 g of hydrogenated castor oil (Cutina HR®) and 500 g of octyl palmitate were introduced into a steel turbine mixer (from Dumec) and stirred while heating to a temperature of approx. 80-90°C until the hydrogenated castor oil was dissolved.

At this point, while stirring was continued and at the same temperature as above, 1500 g of vitamin E acetate were added and a vacuum was applied within the mixer (vacuum of 600 cmHg).

Once the desired level of vacuum had been achieved, 500 g of a previously prepared 8:2 cyclomethicone pentamer/dimethiconol mixture and 1975 g of cyclomethicone pentamer 245 were added while stirring was continued.

The resultant homogeneous mixture was adjusted to ambient temperature while being stirred continuously, finally giving rise to a translucent hydrophobic gel of a semi-solid consistency.

EP 2 334 186 B1

EXAMPLE 3

| d,l-alpha-Tocopherol acetate | 10% |
| Cyclomethicone pentamer | 90% |

The solution obtained by dispersing the alpha-tocopherol acetate in the cyclomethicone was introduced into the collapsible container 1, as described in example 1 and a spray device containing the above formulation was manufactured in the same way as for example 1.

EXAMPLE 4

| d,l-alpha-Tocopherol acetate | 5% |
| Cyclomethicone pentamer | 95% |

The solution obtained by dispersing the alpha-tocopherol acetate in the cyclomethicone was introduced into the collapsible container 1, as described in example 1 and a spray device containing the above formulation was manufactured in the same way as for example 1.

EXAMPLE 5

| d,l-alpha-Tocopherol acetate | 1% |
| Cyclomethicone pentamer | 99% |

The solution obtained by dispersing the alpha-tocopherol acetate in the cyclomethicone was introduced into the collapsible container 1, as described in example 1 and a spray device containing the above formulation was manufactured in the same way as for example 1.

The formulations stated in Examples 1 and 2 were tested for their ability to control head louse infestations (*Pediculus* capitis) as explained below.

TEST 1

Lice were obtained from the heads of volunteer children by being combed from the children's heads in accordance with "Insect R&D Limit Standard Operating Procedure CT.LLI.COM.I" edition no. 1.0. The lice used in the test were accordingly obtained from different sources. This ensured an accurate representation of the normal variation in head lice which may with good probability be encountered in the community, and any variation in response was thus representative of the range of response likely to be encountered in use by the consumer.

The lice were distributed in groups of twenty onto squares of open mesh nylon gauze as a support substrate. Each group was placed on 90 millimetre plastic Petri dish.

Each square (4 cm$^2$) of gauze with the lice attached was then massaged with approx. 5 ml of the formulation to be tested. This was done on the dry gauze so that the formulations could be used neat. The formulations were then left exposed to the air for the specified exposure time.

After the lice had been exposed to the formulation to be tested for a given exposure time, water was added to the gauze, together with approx. 1 ml of shampoo and the gauze was massaged once more to produce a satisfactory foam. The gauze was then rinsed three times with 250 ml of warm tap water (34°C) poured over and through the gauze squares. The gauze was then blotted dry with medical wipe tissue and incubated under normal maintenance conditions in clean plastic Petri dishes of appropriate dimensions until the results were recorded.

The procedure was repeated twice when the spray formulation of Example 1 was used, once with an exposure time of twenty minutes and once with continuous exposure, in which the formulation was not washed away.

When the formulation of Example 2 was used, an exposure time of twenty minutes was used.

A control was also carried out by treating with water, again with an exposure time of 20 minutes.

5

After having washed away the formulations to be tested, the lice were observed at intervals in order to identify any signs of recovery or degeneration. Activity and survival readings were carried out at 1, 2, 3 and 24 hours.

The results are summarized in Table 1 below, which shows the mortality of 20 lice after treatment with the two formulations and with one control.

**TABLE 1**

| Treatment | Time | Total | Dead | Alive | Morbid | Mortality (%) | Corrected mortality (%) |
|---|---|---|---|---|---|---|---|
| **Example 1 formulation (exposure for the entire observation period)** | 1 hour | 20 | 20 | 0 | 0 | 100 | 100 |
| | 2 hours | 20 | 20 | 0 | 0 | 100 | 100 |
| | 3 hours | 20 | 20 | 0 | 0 | 100 | 100 |
| | 24 hours | 20 | 20 | 0 | 0 | 100 | 100 |
| **Example 1 formulation (20 minutes' exposure)** | 1 hour | 20 | 20 | 0 | 0 | 100 | 100 |
| | 2 hours | 20 | 20 | 0 | 0 | 100 | 100 |
| | 3 hours | 20 | 20 | 0 | 0 | 100 | 100 |
| | 24 hours | 20 | 20 | 0 | 0 | 100 | 100 |
| **Example 2 formulation** | 1 hour | 20 | 20 | 0 | 0 | 100 | 100 |
| | 2 hours | 20 | 20 | 0 | 0 | 100 | 100 |
| | 3 hours | 20 | 20 | 0 | 0 | 100 | 100 |
| | 24 hours | 20 | 20 | 0 | 0 | 100 | 100 |
| | 1 hour | 20 | 0 | 100 | 0 | 0 | 0 |
| | 2 hours | 20 | 0 | 100 | 0 | 0 | 0 |
| | 3 hours | 20 | 0 | 100 | 0 | 0 | 0 |
| **Control** | 24 hours | 20 | 0 | 100 | 0 | 0 | 0 |

Corrected mortality was calculated using Abbott's formula which is applied to experimental data obtained from tests using living organisms. The correction takes account of the mortality occurring in the control group of organisms due to factors such as natural mortality and handling procedures.

The formula is as follows:

$$M^1 = \frac{(M^t - M^c)}{(100 - M^c)} \times 100$$

in which:

$M^1$ = corrected mortality
$M^t$ = observed mortality in test group
$M^c$ = observed mortality in control group.

This correction was developed to take account of relatively high mortality levels in control groups of the order of 20% or higher. However, it may be applied to any experiment where it is considered that the mortality of the controls might have an influence on the interpretation of the results.

In some experiments, in which the activity of the substance to be tested is low, the corrected mortality may be less than zero. Results of this type are identified by mortality values enclosed in square brackets with the number preceded by a minus sign, for example [-2.4].

As can be seen from the table, complete mortality of all the lice treated with the formulation according to Example 1 was obtained, both after an exposure time of 20 minutes and with continuous exposure. The results also show mortality of all the lice treated with the formulation of Example 3 after 20 minutes' exposure. All the lice, in all three treatments, were dead within the first hour of exposure to the formulation.

It is possible to rule out that the massaging carried out with the formulations on the gauze contributed to louse mortality, since the control results indicate that the massaging has no effect.

From observing the lice treated with the spray formulation according to Example 1, it was found that the lice subjected to the action of the spray on the flat surface of the Petri dish were enveloped in a spray meniscus and were very rapidly immobilized.

In conclusion, it may be inferred from the table that all the treatments with the two formulations according to the invention led to a 100% increase in louse mortality relative to the control. In every case, 100% mortality was achieved within the first hour after having washed away the formulations under investigation (one hour and twenty minutes after application).

TEST 2

A comparison test was furthermore carried out between the formulation of Example 1, the commercial product HEDRIN (4% solution of dimethicone in cyclomethicone) and a control composed of water.

Head lice were obtained from volunteer children using the same procedures outlined above for test 1.

The lice were distributed in groups of twenty onto squares of open mesh nylon gauze as a support substrate. Each group was placed on 90 millimetre plastic Petri dish.

Each square (4 cm$^2$) of gauze with the lice attached was then massaged with approx. 5 ml of the formulation to be tested. This was done on the dry gauze so that the formulations could be used neat. The formulations were then left exposed to the air for the specified exposure time.

After the lice had been exposed to the formulation to be tested for a given exposure time, water was added to the gauze, together with approx. 1 ml of shampoo and the gauze was massaged once more to produce a satisfactory foam. The gauze was then rinsed three times with 250 ml of warm tap water (34°C) poured over and through the gauze squares. The gauze was then blotted dry with medical wipe tissue and incubated under normal maintenance conditions in clean plastic Petri dishes of appropriate dimensions until the results were recorded.

The procedure was carried out three times using the formulation of Example 1, with an exposure time of 20 minutes, and three times using the product HEDRIN, again with a exposure time of 20 minutes.

Once rinsing had been carried out as above, the lice were observed at specified intervals in order to ascertain any signs of recovery or degeneration. Activity and survival observations were carried out after 1, 2, 3 and 24 hours had elapsed.

The results of the observations are summarized in Tables 2, 3 and 4 below.

TABLE 2

| Treatment | Time | Total | Dead | Alive | Morbid | Mortality (%) | Corrected mortality (%) |
|---|---|---|---|---|---|---|---|
| Example 1 formulation (20 minutes' exposure) | 1 hour | 20 | 20 | 0 | 0 | 100 | 100 |
| | 2 hours | 20 | 20 | 0 | 0 | 100 | 100 |
| | 3 hours | 20 | 20 | 0 | 0 | 100 | 100 |
| | 24 hours | 20 | 20 | 0 | 0 | 100 | 100 |
| Example 1 formulation (20 minutes' exposure) | 1 hour | 20 | 20 | 0 | 0 | 100 | 100 |
| | 2 hours | 20 | 20 | 0 | 0 | 100 | 100 |
| | 3 hours | 20 | 20 | 0 | 0 | 100 | 100 |
| | 24 hours | 20 | 20 | 0 | 0 | 100 | 100 |
| Example 1 formulation (20 minutes' exposure) | 1 hour | 20 | 20 | 0 | 0 | 100 | 100 |
| | 2 hours | 20 | 20 | 0 | 0 | 100 | 100 |
| | 3 hours | 20 | 20 | 0 | 0 | 100 | 100 |
| | 24 hours | 20 | 20 | 0 | 0 | 100 | 100 |

Table 2 shows mortality data for three groups of 20 lice after treatment with the formulation according to Example 1.

TABLE 3

| Treatment | Time | Total | Dead | Alive | Morbid | Mortality (%) | Corrected mortality (%) |
|---|---|---|---|---|---|---|---|
| | 1 hour | 20 | 20 | 0 | 0 | 100 | 100 |
| | 2 hours | 20 | 20 | 0 | 0 | 100 | 100 |
| | 3 hours | 20 | 20 | 0 | 0 | 100 | 100 |
| HEDRIN | 24 hours | 20 | 20 | 0 | 0 | 100 | 100 |
| | 1 hour | 20 | 16 | 1 | 3 | 95 | 94.0 |
| | 2 hours | 20 | 20 | 0 | 0 | 100 | 100 |
| | 3 hours | 20 | 20 | 0 | 0 | 100 | 100 |
| HEDRIN | 24 hours | 20 | 20 | 0 | 0 | 100 | 100 |
| | 1 hour | 20 | 15 | 4 | 1 | 80 | 76.01 |
| | 2 hours | 20 | 16 | 3 | 1 | 85 | 80.86 |
| | 3 hours | 20 | 18 | 1 | 1 | 95 | 93.18 |
| HEDRIN | 24 hours | 20 | 18 | 1 | 1 | 95 | 40.05 |

Table 3 shows the mortality of three groups of 20 lice after treatment with HEDRIN.

TABLE 4

| Treatment | Time | Total | Dead | Alive | Morbid | Mortality (%) | Corrected mortality (%) |
|---|---|---|---|---|---|---|---|
| | 1 hour | 20 | 4 | 14 | 2 | 30 | N/A |
| | 2 hours | 20 | 7 | 11 | 2 | 45 | N/A |
| | 3 hours | 20 | 10 | 8 | 2 | 60 | N/A |
| Control (water) | 24 hours | 20 | 16 | 3 | 1 | 85 | N/A |
| | 1 hour | 20 | 0 | 20 | 0 | 0 | N/A |
| | 2 hours | 20 | 0 | 20 | 0 | 0 | N/A |
| | 3 hours | 20 | 0 | 20 | 0 | 0 | N/A |
| Control (water) | 24 hours | 20 | 16 | 2 | 2 | 90 | N/A |
| | 1 hour | 20 | 3 | 16 | 1 | 20 | N/A |
| | 2 hours | 20 | 3 | 16 | 1 | 20 | N/A |
| | 3 hours | 20 | 3 | 16 | 1 | 20 | N/A |
| Control (water) | 24 hours | 20 | 20 | 0 | 0 | 100 | N/A |
| N/A = Not applicable | | | | | | | |

Table 4 shows the mortality of three groups of 20 lice after treatment with water.

As can be seen from Table 2, treatment with the formulation according to Example 1 brought about complete mortality of all the lice after an exposure time of 20 minutes. In all three of the treated groups, all the lice were observed to die within the first hour following exposure to the formulation and no signs of recovery were observed after 2, 3 and 24 hours had elapsed.

In general, treatment with the formulation according to Example 1 proved more effective than treatment with the comparison product, HEDRIN, even if this latter product also exhibited a significant increase in mortality relative to the controls.

However, in one of the groups treated with HEDRIN, it took two hours from the washing treatment to achieve the death of all the lice. In another group, two lice even remained alive after 24 hours.

TEST 3

A comparison test was also carried out between the formulation of Example 1, a compressed air spray similar to that described in Example 1, but containing 100% of cyclomethicone and a control composed of water.

Head lice were obtained from volunteer children using the same procedures outlined above for test 1.

The lice were distributed in groups of twenty onto squares of open mesh nylon gauze as a support substrate. Each group was placed on 90 millimetre plastic Petri dish.

Each square (4 cm$^2$) of gauze with the lice attached was then massaged with approx. 5 ml of the formulation to be tested. This was done on the dry gauze so that the formulations could be used neat. The formulations were then left exposed to the air for the specified exposure time.

After the lice had been exposed to the formulation to be tested for a given exposure time, water was added to the gauze, together with approx. 1 ml of shampoo and the gauze was massaged once more to produce a satisfactory foam. The gauze was then rinsed three times with 250 ml of warm tap water (34°C) poured over and through the gauze squares. The gauze was then blotted dry with medical wipe tissue and incubated under normal maintenance conditions in clean plastic Petri dishes of appropriate dimensions until the results were recorded.

The procedure was carried out three times using the formulation of Example 1, with an exposure time of 20 minutes, and three times using the spray containing 100% cyclomethicone, again with a exposure time of 20 minutes. The procedure was also carried out three times with the control, carrying out treatment with water, in this case too with an exposure time of 20 minutes.

Once rinsing had been carried out as above, the lice were observed at specified intervals in order to ascertain any signs of recovery or degeneration. Activity and survival observations were carried out after 1, 2, 3 and 24 hours had elapsed.

The results of the observations are summarized in tables 5, 6 and 7 below.

Table 5

| Treatment | Time | Total | Dead | Alive | Morbid | Mortality (%) | Corrected mortality (%) |
|---|---|---|---|---|---|---|---|
| 100% cyclomethicone spray (20 minutes' exposure) | 1 hour | 20 | 20 | 0 | 0 | 100 | 100 |
| | 2 hours | 20 | 20 | 0 | 0 | 100 | 100 |
| | 3 hours | 20 | 19 | 0 | 1 | 100 | 100 |
| | 24 hours | 20 | 19 | 0 | 1 | 100 | 100 |
| 100% cyclomethicone spray (20 minutes' exposure) | 1 hour | 20 | 20 | 0 | 0 | 100 | 100 |
| | 2 hours | 20 | 20 | 0 | 0 | 100 | 100 |
| | 3 hours | 20 | 20 | 0 | 0 | 100 | 100 |
| | 24 hours | 20 | 17 | 2 | 1 | 90 | 86.36 |
| 100% cyclomethicone spray (20 minutes' exposure) | 1 hour | 20 | 20 | 0 | 0 | 100 | 100 |
| | 2 hours | 20 | 20 | 0 | 0 | 100 | 100 |
| | 3 hours | 20 | 18 | 1 | 1 | 95 | 93.18 |
| | 24 hours | 20 | 14 | 4 | 2 | 80 | [-1.4] |

Table 5 shows the mortality of three groups of 20 lice after treatment with 100% cyclomethicone spray.

Table 6

| Treatment | Time | Total | Dead | Alive | Morbid | Mortality (%) | Corrected mortality (%) |
|---|---|---|---|---|---|---|---|
| Example 1 formulation (20 minutes' exposure) | 1 hour | 20 | 20 | 0 | 0 | 100 | 100 |
| | 2 hours | 20 | 20 | 0 | 0 | 100 | 100 |
| | 3 hours | 20 | 20 | 0 | 0 | 100 | 100 |
| | 24 hours | 20 | 20 | 0 | 0 | 100 | 100 |
| Example 1 formulation (20 minutes' exposure) | 1 hour | 20 | 20 | 0 | 0 | 100 | 100 |
| | 2 hours | 20 | 20 | 0 | 0 | 100 | 100 |
| | 3 hours | 20 | 20 | 0 | 0 | 100 | 100 |
| | 24 hours | 20 | 20 | 0 | 0 | 100 | 100 |

(continued)

| Treatment | Time | Total | Dead | Alive | Morbid | Mortality (%) | Corrected mortality (%) |
|---|---|---|---|---|---|---|---|
| **Example 1 formulation (20 minutes' exposure)** | 1 hour | 20 | 20 | 0 | 0 | 100 | 100 |
| | 2 hours | 20 | 20 | 0 | 0 | 100 | 100 |
| | 3 hours | 20 | 20 | 0 | 0 | 100 | 100 |
| | 24 hours | 20 | 20 | 0 | 0 | 100 | 100 |

Table 6 shows mortality data for three groups of 20 lice after treatment with the formulation according to Example 1.

Table 7

| Treatment | Time | Total | Dead | Alive | Morbid | Mortality (%) | Corrected mortality (%) |
|---|---|---|---|---|---|---|---|
| **Control (water)** | 1 hour | 20 | 4 | 14 | 2 | 30 | N/A |
| | 2 hours | 20 | 7 | 11 | 2 | 45 | N/A |
| | 3 hours | 20 | 10 | 8 | 2 | 60 | N/A |
| | 24 hours | | 16 | 3 | 1 | 85 | N/A |
| **Control (water)** | 1 hour | 20 | 0 | 20 | 0 | 0 | N/A |
| | 2 hours | 20 | 0 | 20 | 0 | 0 | N/A |
| | 3 hours | 20 | 0 | 20 | 0 | 0 | N/A |
| | 24 hours | 20 | 16 | 2 | 2 | 90 | N/A |
| **Control (water)** | 1 hour | 20 | 3 | 16 | 1 | 20 | N/A |
| | 2 hours | 20 | 3 | 16 | 1 | 20 | N/A |
| | 3 hours | 20 | 3 | 16 | 1 | 20 | N/A |
| | 24 hours | 20 | 20 | 0 | 0 | 100 | N/A |
| N/A = Not applicable. | | | | | | | |

Table 7 shows the mortality of three groups of 20 lice after treatment with water.

It may be seen from Table 5 that the 100% cyclomethicone spray brought about 100% mortality in two of the groups tested after 3 hours, but a mortality of 95% in the third group, which results in a mean mortality of 98.33%, which is 71.67% higher than that found for the controls. All the lice in all three groups appeared to be dead after 1 hour, however a certain level of recovery was observed in all three groups and, after 24 hours, mean mortality was 90%.

The results in Table 6 show 100% mortality of all the lice treated with the formulation according to Example 1, after an exposure time of 20 minutes. All the lice, in all three groups, were dead within the first hour after exposure to the formulation. No recovery of the lice was observed. The formulation according to Example 1 brought about a mortality rate after 3 hours which was 73.34% higher than that of the controls and 1.67% higher than that observed for the 100% cyclomethicone spray.

After 24 hours, the formulation according to Example 1 brought about a mortality rate 10% higher than that found with the 100% cyclomethicone spray. This clearly demonstrates that the vitamin E acetate present in the formulation according to Example 1 has a significant effect on the lice.

TEST 4

The activity of the formulation according to the present invention was tested at the three different dose levels of Examples 3 to 5.

Head lice were obtained from volunteer children using the same procedures outlined above for test 1.

The lice were distributed in groups of fifteen onto squares of open mesh nylon gauze as a support substrate. Each group was placed on 90 millimetre plastic Petri dish.

Each square (4 cm$^2$) of gauze with the lice attached was then massaged with approx. 5 ml of the formulation to be tested. This was done on the dry gauze so that the formulations could be used neat. The formulations were then left exposed to the air for the specified exposure time.

After the lice had been exposed to the formulation to be tested for ten minutes, water was added to the gauze, together with approx. 1 ml of shampoo and the gauze was massaged once more to produce a satisfactory foam. The gauze was then rinsed three times with 250 ml of warm tap water (34°C) poured over and through the gauze squares. The gauze was then blotted dry with medical wipe tissue and incubated under normal maintenance conditions in clean plastic Petri dishes of appropriate dimensions until the results were recorded.

Once rinsing had been carried out as above, the lice were observed at specified intervals in order to ascertain any signs of recovery or degeneration. Activity and survival observations were carried out after 1, 2, 3 and 24 hours had elapsed.

A control was also carried out on a group of 12 lice by treating with water, again with an exposure time of 10 minutes.

The results of the observations are summarized in Tables 8 and 9 below.

TABLE 8

| Treatment | Time | Total | Dead | Alive | Morbid | Mortality (%) |
|---|---|---|---|---|---|---|
| | 1 hour | 15 | 15 | 0 | 0 | 100 |
| | 2 hours | 15 | 15 | 0 | 0 | 100 |
| | 3 hours | 15 | 15 | 0 | 0 | 100 |
| **Example 3 formulation** | 24 hours | 15 | 15 | 0 | 0 | 100 |
| | 1 hour | 15 | 15 | 0 | 0 | 100 |
| | 2 hours | 15 | 15 | 0 | 0 | 100 |
| | 3 hours | 15 | 15 | 0 | 0 | 100 |
| **Example 4 formulation** | 24 hours | 15 | 15 | 0 | 0 | 100 |
| | 1 hour | 15 | 15 | 0 | 0 | 100 |
| | 2 hours | 15 | 15 | 0 | 0 | 100 |
| | 3 hours | 15 | 15 | 0 | 0 | 100 |
| **Example 5 formulation** | 24 hours | 15 | 15 | 0 | 0 | 100 |

TABLE 9

| Treatment | Time | Total | Dead | Alive | Morbid | Mortality (%) |
|---|---|---|---|---|---|---|
| | 1 hour | 12 | 0 | 12 | 0 | 0 |
| | 2 hours | 12 | 0 | 12 | 0 | 0 |
| | 3 hours | 12 | 0 | 12 | 0 | 0 |
| **Control (water)** | 24 hours | 12 | 0 | 11 | 1 | 8.3 |

Table 8 shows that the formulations according to the present invention is effective at killing all the lice one hour after they have been washed off. The lice did not recover over the 24 hour period demonstrating 100% efficacy of all three dose levels tested. The control group performed as expected with most lice surviving for 24 hours.

The lice were tested with a ten minute exposure before being washed off; this was to prove the lowest time that they could be exposed to the formulation to give 100% mortality. If the time is increased to twenty minutes then the results would still show 100% efficacy. This demonstrates that if required the contact time can be reduced.

**Claims**

1. Non-therapeutic use of a composition consisting of vitamin E or an ester thereof selected among the group consisting of esters of tocopherol with acetic, linoleic, oleic, palmitic or succinic acid, and a solvent selected from the group consisting of volatile siloxanes, hydrogenated polyisobutene, hydrogenated polydecene and mixtures of hydrogenated polyisobutene and/or hydrogenated polydecene with hydrogenated polyolefines, in the control of ectoparasites.

2. The non-therapeutic use according to claim 1, wherein said solvent is a volatile siloxane selected among cyclomethicone pentamer, cyclomethicone tetramer, hexamethyldisiloxane and mixtures thereof.

3. The non-therapeutic use according to claim 2, wherein said volatile siloxane is cyclomethicone pentamer.

4. The non-therapeutic use according to any one of claims 1 to 3, wherein said ectoparasites are lice.

5. The non-therapeutic use according to any one of the preceding claims, wherein said composition comprises from 1% to 90% of vitamin E or said ester thereof.

6. The non-therapeutic use according to any one of claims 1 to 4, wherein said composition comprises from 1% to 30% of vitamin E or said ester thereof.

7. The non-therapeutic use according to any one of the preceding claims, wherein said composition consists of vitamin E or said ester thereof and a volatile siloxane.

8. The non-therapeutic use according to claim 7, wherein said composition consists of vitamin E acetate and cyclomethicone pentamer.

9. The non-therapeutic use according to claim 7 or 8, wherein said composition is delivered by means of a spray delivery device.

10. The non-therapeutic use according to claim 9, wherein said device comprises a collapsible container (1) which is in turn enclosed in a spray can (4) filled with a pressurized fluid which causes said composition to be delivered in finely atomized form.

11. Non-therapeutic use of a composition in the form of a hydrophobic gel, comprising vitamin E or an ester thereof, selected among the group consisting of esters of tocopherol with acetic, linoleic, oleic, palmitic or succinic acid; a volatile silicone, selected from the group consisting of cyclomethicone pentamer, cyclomethicone tetramer, cyclomethicone hexamer, hexamethyldisiloxane and mixtures thereof; and hydrogenated castor oil, in the control of ectoparasites.

12. The non-therapeutic use according to claim 11, wherein said composition comprises from 1 to 70% of vitamin E or said ester thereof, from 20 to 90% of said volatile silicone and from 7 to 13% of hydrogenated castor oil.

13. The non-therapeutic use according to claim 11, wherein said composition comprises from 1% to 50% of vitamin E acetate, from 40 to 70% of said volatile silicone and from 7 to 13% of hydrogenated castor oil.

14. The non-therapeutic use according to any one of claims 11 to 13, wherein said volatile silicone is cyclomethicone pentamer.

15. The non-therapeutic use according to any one of claims 11 to 14, wherein said composition further comprises from 7 to 15% of an oily component selected from the group consisting of vegetable oils and esters of fatty acids, and optionally 2 to 3% of dimethiconol.

16. The non-therapeutic use according to claim 15, wherein said oily component consists of octyl palmitate, isopropyl myristate, ethyl oleate and mixtures thereof.

**Patentansprüche**

1. Nicht-therapeutische Verwendung einer Zusammensetzung bestehend aus Vitamin E oder einem Ester aus diesem, ausgewählt aus der Gruppe bestehend aus Tocopherol-Estern mit Essigsäure, Linolsäure, Ölsäure, Palmitin- oder Bernsteinsäure, und einem Lösungsmittel, ausgewählt aus der Gruppe bestehend aus flüchtigen Siloxanen, hydriertem Polyisobuten, hydrierten Polydecenen und Mischungen aus hydriertem Polyisobuten und/oder hydrierten Polydecenen mit hydrierten Polyolefinen, zur Bekämpfung von Ektoparasiten.

2. Nicht-therapeutische Verwendung nach Anspruch 1, wobei das Lösungsmittel ein flüchtiges Siloxan ist, ausgewählt aus einem Cyclomethicone-Pentamer, einem Cyclomethicone-Tetramer, Hexamethyldisiloxan und Mischungen aus diesen.

3. Nicht-therapeutische Verwendung nach Anspruch 2, wobei das flüchtige Siloxan ein Cyclometicone-Pentamer ist.

4. Nicht-therapeutische Verwendung nach einem der Ansprüche 1 bis 3, wobei die Ektoparasiten Läuse sind.

5. Nicht-therapeutische Verwendung nach einem der vorhergehenden Ansprüche, wobei die Zusammensetzung zu einem Anteil von 1% bis 90% aus Vitamin E oder dem Ester aus diesem besteht.

6. Nicht-therapeutische Verwendung nach einem der Ansprüche 1 bis 4, wobei die Zusammensetzung zu einem Anteil von 1 % bis 30% aus Vitamin E oder dem Ester aus diesem besteht.

7. Nicht-therapeutische Verwendung nach einem der vorhergehenden Ansprüche, wobei die Zusammensetzung aus Vitamin E oder dem Ester aus diesem und einem flüchtigen Siloxan besteht.

8. Nicht-therapeutische Verwendung nach Anspruch 7, wobei die Zusammensetzung aus Vitamin-E-Acetat und einem Cyclomethicone-Pentamer besteht.

9. Nicht-therapeutische Verwendung nach Anspruch 7 oder 8, wobei die Zusammensetzung mittels einer Sprühvorrichtung abgegeben wird.

10. Nicht-therapeutische Verwendung nach Anspruch 9, wobei die Vorrichtung einen zusammenklappbaren Behälter (1) umfasst, der wiederum in einer Sprühdose (4) untergebracht ist, die ein unter Druck stehendes Fluid enthält, wodurch die Zusammensetzung in fein zerstäubter Form abgegeben wird.

11. Nicht-therapeutische Verwendung einer Zusammensetzung in Form eines hydrophoben Gels, umfassend Vitamin E oder ein Ester aus diesem, ausgewählt aus der Gruppe bestehend aus Tocopherol-Estern mit Essigsäure, Linolsäure, Ölsäure, Palmitin- oder Bernsteinsäure, ein flüchtiges Silikon, ausgewählt aus der Gruppe bestehend aus einem Cyclomethicone-Pentamer, einem Cyclomethicone-Tetramer, einem Cyclomethicone-Hexamer, Hexamethyldisiloxan und Mischungen aus diesen, und ein hydriertes Kastoröl, zur Bekämpfung von Ektoparasiten.

12. Nicht-therapeutische Verwendung nach Anspruch 11, wobei die Zusammensetzung zu einem Anteil von 1% bis 70% aus Vitamin E oder dem Ester aus diesem, zu einem Anteil von 20% bis 90% aus dem flüchtigen Silikon und zu einem Anteil von 7% bis 13% aus hydriertem Castoröl besteht.

13. Nicht-therapeutische Verwendung nach Anspruch 11, wobei die Zusammensetzung zu einem Anteil von 1 % bis 50% aus Vitamin-E-Acetat, zu einem Anteil von 40% bis 70% aus dem flüchtigen Silikon und zu einem Anteil von 7% bis 13% aus hydriertem Kastoröl besteht.

14. Nicht-therapeutische Verwendung nach einem der Ansprüche 11 bis 13, wobei das flüchtige Silikon ein Cyclomethicone-Pentamer ist.

15. Nicht-therapeutische Verwendung nach einem der Ansprüche 11 bis 14, wobei die Zusammensetzung ferner zu einem Anteil von 7% bis 15% eine Öl-Komponente umfasst, die aus der Gruppe bestehend aus Pflanzenölen und Fettsäure-Estern ausgewählt ist, und optional zu einem Anteil von 2% bis 3% aus Dimethiconol besteht.

16. Nicht-therapeutische Verwendung nach Anspruch 15, wobei die Öl-Komponente aus Octylpalmitat, Isopropylmyristat, Ethyloleat und Mischungen aus diesen besteht.

**Revendications**

1. Utilisation non thérapeutique d'une composition constituée de vitamine E ou d'un ester de celle-ci choisi dans le groupe constitué par les esters de tocophérol avec l'acide acétique, linoléique, oléique, palmitique ou succinique, et d'un solvant choisi dans le groupe constitué par les siloxanes volatils, le polyisobutène hydrogéné, le polydécène hydrogéné et les mélanges de polyisobutène hydrogéné et/ou de polydécène hydrogéné avec des polyoléfines hydrogénées, pour la lutte contre les ectoparasites.

**2.** Utilisation non thérapeutique selon la revendication 1, dans laquelle ledit solvant est un siloxane volatil choisi parmi la cyclométhicone pentamère, la cyclométhicone tétramère, l'hexaméthyldisiloxane, et leurs mélanges.

**3.** Utilisation non thérapeutique selon la revendication 2, dans laquelle ledit siloxane volatil est la cyclométhicone pentamère.

**4.** Utilisation non thérapeutique selon l'une quelconque des revendications 1 à 3, dans laquelle lesdits ectoparasites sont des poux.

**5.** Utilisation non thérapeutique selon l'une quelconque des revendications précédentes, dans laquelle ladite composition comprend de 1 % à 90 % de vitamine E ou dudit ester de celle-ci.

**6.** Utilisation non thérapeutique selon l'une quelconque des revendications 1 à 4, dans laquelle ladite composition comprend de 1 % à 30 % de vitamine E ou dudit ester de celle-ci.

**7.** Utilisation non thérapeutique selon l'une quelconque des revendications précédentes, dans laquelle ladite composition est constituée de vitamine E ou dudit ester de celle-ci et d'un siloxane volatil.

**8.** Utilisation non thérapeutique selon la revendication 7, dans laquelle ladite composition est constituée d'acétate de vitamine E et de cyclométhicone pentamère.

**9.** Utilisation non thérapeutique selon la revendication 7 ou 8, dans laquelle ladite composition est délivrée au moyen d'un dispositif de délivrance par pulvérisation.

**10.** Utilisation non thérapeutique selon la revendication 9, dans laquelle ledit dispositif comprend un récipient compressible (1) qui est à son tour enfermé dans une bombe aérosol (4) remplie d'un fluide pressurisé qui provoque la délivrance de ladite composition sous une forme finement atomisée.

**11.** Utilisation non thérapeutique d'une composition sous la forme d'un gel hydrophobe, comprenant de la vitamine E ou un ester de celle-ci, choisi dans le groupe constitué par les esters de tocophérol avec l'acide acétique, linoléique, oléique, palmitique ou succinique ; une silicone volatile, choisie dans le groupe constitué par la cyclométhicone pentamère, la cyclométhicone tétramère, la cyclométhicone hexamère, l'hexaméthyldisiloxane et leurs mélanges ; et de l'huile de ricin hydrogénée, dans la lutte contre les ectoparasites.

**12.** Utilisation non thérapeutique selon la revendication 11, dans laquelle ladite composition comprend de 1 à 70 % de vitamine E ou dudit ester de celle-ci, de 20 à 90 % de ladite silicone volatile et de 7 à 13 % d'huile de ricin hydrogénée.

**13.** Utilisation non thérapeutique selon la revendication 11, dans laquelle ladite composition comprend de 1 à 50 % d'acétate de vitamine E, de 40 à 70 % de ladite silicone volatile et de 7 à 13 % d'huile de ricin hydrogénée.

**14.** Utilisation non thérapeutique selon l'une quelconque des revendications 11 à 13, dans laquelle ladite silicone volatile est la cyclométhicone pentamère.

**15.** Utilisation non thérapeutique selon l'une quelconque des revendications 11 à 14, dans laquelle ladite composition comprend en outre de 7 à 15 % d'un composant huileux choisi dans le groupe constitué par les huiles végétales et les esters d'acides gras, et éventuellement 2 à 3 % de diméthiconol.

**16.** Utilisation non thérapeutique selon la revendication 15, dans laquelle ledit composant huileux est constitué de palmitate d'octyle, de myristate d'isopropyle, d'oléate d'éthyle et de leurs mélanges.

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 1604853 A
- EP 0191543 A
- EP 1215965 A
- DE 19622190
- FR 2436085

**Non-patent literature cited in the description**

- *British Medical Journal,* 2005, vol. 330, 1423
- Insect R&D Limit Standard Operating Procedure CT.LLI.COM.I